# EUROPEAN PATENT SPECIFICATION

(11) **EP 4 333 976 B1**
(45) Date of publication and mention of the grant of the patent: **14.05.2025**
(21) Application number: 22722359.1
(22) Date of filing: 02.05.2022
(51) Int. Cl.: A61N 1/40, A61N 5/10

(54) **INTERSTITIAL HYPERTHERMIA DEVICE**
INTERSTITIELLE HYPERTHERMIEVORRICHTUNG
DISPOSITIF D'HYPERTHERMIE INTERSTITIELLE

(30) Priority: 03.05.2021 NL 2028131
(43) Date of publication of application: 13.03.2024
(73) Proprietor: Erasmus University Medical Center Rotterdam, 3015 GE Rotterdam (NL); Technische Universiteit Eindhoven, 5612 AE Eindhoven (NL)
(72) Inventor: ANDROULAKIS, Ioannis, 3015 GE Rotterdam (NL); DEURLOO, Inger Karine Kirsten, 3015 GE Rotterdam (NL); VAN RHOON, Gerard Cornelis, 3015 GE Rotterdam (NL); CHRISTIANEN, Miranda Eligia Maria Cornelia, 3015 GE Rotterdam (NL); MESTROM, Robertus Matheus Catharina, 5612 AE Eindhoven (NL)
(74) Representative: V.O.
(86) International application number: PCT/NL2022/050237
(87) International publication number: WO 2022/235155

(56) References cited:
- WO-A1-2014/064552
- US-A- 5 531 662
- US-A- 5 620 479
- US-A1- 2004 243 200
- US-A1- 2005 251 235

## Description

### Field of the invention

High Dose Rate Brachytherapy (HDR BT) is intensively used as an exclusive modality for the treatment of intermediate and low risk prostate cancer. It is also used in combination with external beam radiotherapy for the treatment of high-risk prostate cancer. Accumulating evidence shows that prostate cancer can be hypofractionated (higher dose in less fractions) without losing treatment efficiency compared to normal fractionation schedules. The drawback of hypofractionation, especially with BT, is the risk of radiation-induced toxicity of critical structures in close proximity to the prostate, including the rectum wall, the bladder and the urethra. This toxicity is also a reason that HDR BT is not implemented in high risk prostate cancer patients as a monotherapy.

With hyperthermia being recognized as the strongest radiosensitizing agent currently available, with limited or no toxicity for normal structures when the heat delivery is carefully monitored, combining ultra-hypofractionated HDR with interstitial hyperthermia could lead to a further improvement of prostate cancer treatment.

The radiosensitizing effect of hyperthermia is known to be highest when hyperthermia is applied simultaneously with the radiotherapy treatment. However, current interstitial hyperthermia solutions are not optimal in terms of efficiency and furthermore do not allow for simultaneous HDR BT. Therefore, a need exists for the development of a system that would be able to simultaneously and seamlessly integrate a well-controlled and localized hyperthermia treatment in the existing HDR-BT workflow.

### Background of the invention

In (prostate) cancer treatment, HDR BT can be applied interstitially, commonly through 6F (2 mm OD) thermoplastic afterloading catheters inserted into the target volume. The afterloading catheters are connected to the afterloader, so that a radioactive source can sequentially move to each dwell position inside the target area for a pre-planned dwell time. This process may take approximately 10-20 minutes.

For the thermal enhancement of the brachytherapy treatment, hyperthermia can be applied interstitially (IHT) as well. This ensures better control and localization of the thermal distribution compared to other hyperthermia techniques that heat using an external source.

Currently, Microwave antenna (MW) based IHT is the most common type of IHT. In one approach, flexible MW antennas may be used together with 6F catheters used for HDR-BT. However, the MW-frequency used prescribes the length of the heating pattern. Hence in clinical practice MW-IHT is seriously compromised as heating length cannot be chosen freely and longitudinal temperature steering is not possible. Also, a heating pattern is usually ellipsoidal, with the maximum at the center and low values at the tip which cannot be altered.

Another approach is the use of Local Current Field Radio Frequency (RF) electrodes. However, the temperature distribution becomes very sensitive to the tissue properties, and real-time adaptive longitudinal steering of the thermal dose pattern is impossible.

A yet further approach is heating by capacitive coupling . This type of IHT may deliver a homogenous longitudinal temperature distribution. In this case a quasi-static electric field is applied that interacts with the tissue and by having the electric field alternate in time, a capacitive coupling is provided that heats the tissue. The electric field is quasi-static in that it interacts by frequencies of e.g. max 50 MHz that interact with polarized molecules and thereby heat the tissue, and having no or minimal radiative effect to be able to precisely control the heating zone, which may typically be a target of about 1-10 cm cross section.

While in the art attempts have been made to provide for simultaneous hyperthermia and brachytherapy, clinical use appeared until thus far considerably hampered by larger implant diameters and temperature control that require adaptation of current state-of-art remote HDR-BT hardware. As a consequence these limitations have nullified their clinical use and the ability to apply simultaneous thermo-brachytherapy is only academic and never made it to clinical application.

A device according to the pre-ampble of claim 1 is known from WO-A-2014/064552.

It is an object of the present invention to further advance the field of interstitial hyperthermia device to address these and other challenges.

### Summary of the invention

According to one aspect, an interstitial hyperthermia device has an electrode structure to be coupled to an electric power source for providing an alternating electric field for heating up a patient's tissue. The device is provided with a hollow source guide for conducting a radiation source capsule to be moved by a guidewire. The hollow source guide has an inner wall for guiding the source capsule and an outer wall to be contacted with the patient's tissue. The outer wall is provided with the electrode structure arranged on an outer wall of the hollow source guide and having a dielectric layer shielding the electrode structure from the patient's tissue.

The device according to the invention enables well-localized deposition of energy, can easily combine with current state-of-art clinical HDR-BT routine practice, preferentially utilizing the exact same catheters. Furthermore, the efficiency is optimized by increasing the penetration depth in the tissue, preventing possible overheating of the catheter wall. By utilizing a dielectric layer of the type mentioned, the applicator device can simultaneously provide a well-distributed and controlled hyperthermia thermal dose, and serve as a BT source guide, since electric losses in the layer are minimal, thus preventing heat up by deposition of energy in the catheter walls. A much higher efficiency than earlier IHT devices is obtained by avoiding a relatively thick dielectric layer between the electrode and the tissue, ensuring that the energy is deposited in the tissue rather than the applicator. This is accomplished by the outer wall being provided with an electrode structure arranged on an outer wall and having a dielectric layer shielding the electrode structure from the patient's tissue thereby placing the electrodes coated with a thin dielectric layer directly on the outer side of the source guide. A considerably higher thermal enhancement of the radiation dose, and an online highly adaptable biological effective dose (BED) planning are possible for optimal targeting.

The principle of heating thus shifts towards absorption of electromagnetic energy in tissue directly, instead of heating by thermal conduction from the heated catheter wall to the tissue.

### BRIEF DESCRIPTION OF THE DRAWINGS

The invention will be further elucidated in the figures:
- Figure 1 shows a cross sectional view of the interstitial hyperthermia device
- Figure 2 shows a perspective view of the device in Figure 1;
- Figure 3 shows an interstitial hyperthermia device with another electrode configuration
- Figure 4 shows electromagnetic and heating simulation results of a single applicator.
- Figure 5 shows an interstitial hyperthermia control system for driving multiple interstitial devices simultaneously
- Figure 6 shows a temperature profile of an electrode structure in a number of sequentially driven control modes
- Figure 7 shows a treatment plan system that is provided to the interstitial hyperthermia control system
- Figure 8 (A and B) shows a further detail of an exemplary embodiment;
- Figure 9 (A and B) shows an alternative embodiment, having an asymmetric temperature profile.

### DETAILED DESCRIPTION

Aspects of the invention pertain to an interstitial hyperthermia device having an electrode structure to be coupled to an electric power source for providing an alternating electric field for heating up a patient's tissue; said device provided with a hollow source guide for conducting a radiation source capsule to be moved by a guidewire; said hollow source guide having an inner wall for guiding the source capsule and an outer wall to be contacted with the patients tissue; the outer wall being provided with the electrode structure arranged on a circumference of the outer wall of the hollow source guide and having a dielectric coating layer shielding the electrode structure from the patient's tissue.

In some embodiments the interstitial hyperthermia device has the electrode structure arranged on a polymer tube, that conveniently carries the electrode structure and at the same time provides passage to the radiation source capsule. In preferred embodiments the dielectric layer has a thickness ranging between 20 and 40 micrometers. While dielectric losses are minimal in this range, at the same time it is ensured that no electrical contact is possible with human tissue. Smaller coating thickness could be vulnerable to risks; larger coating thickness could hinder an efficient coupling to the tissue. While in principle, a single electrode could be provided in the device, when cooperating with another device to be driving in antiphase, preferably the interstitial hyperthermia device has an electrode structure that comprises a plurality of electrodes arranged in axial direction along the source guide. The plurality may be two or more, in particular three or four electrodes driven individually along a length of the device. At least one electrode of said plurality of electrodes can be provided with an axial passage, aligned with the tube. The passage may provide a directionality to the thermal profile, e.g. by having covered only a part of the circumference of the electrode, e.g. a part less than 320 degrees, or less than 270 degrees, e.g. ranging from 180 to 320 degrees covering. An electrode wire may be provided running through said passage, that connects to another electrode of said plurality of electrodes. Depending on the number of electrodes, the number of electrodes may vary. This may result in a somewhat asymmetric field, which may have some advantages in the design of a heat profile in the patient's tissue. In preferred embodiments the dielectric coating layer is a Parylene layer, which is a material that is biocompatible, has good dielectric characteristics and can be applied easily in very small layers at ambient temperatures. One or more of the hyperthermia devices can be coupled to a control system programmed to drive one or more electrodes in the electrode structure of one or more of the hyperthermia devices, to create a three-dimensional heat profile in the patient's tissue. The interstitial hyperthermia control system may be programmed to drive said one or more electrodes in an electrode structure of one hyperthermia device in anti-phase relative to one or more electrodes in an electrode structure of another hyperthermia device, when positioned adjacent to each other and separated from each other, in order to create an optimal temperature profile. Depending on the electrode configuration design, a plurality of electrodes may be arranged in axial direction along the source guide and can be driven in antiphase relative to each other, e.g. two by two. A treatment and a treatment planning module may be provided for automation of combined treatment of hyperthermia and brachytherapy. The treatment planning module can be provided with an imaging module; arranged to receive an image of the implanted interstitial hyperthermia device in the patient's tissue to be treated. A target and organ delineation module may be arranged to identify a treatment target and organs at risk in the received image. Process modules may be provided for processing hyperthermia therapy control data in accordance with a treatment plan, and said identified treatment targets and organs at risk; and for controlling brachy therapy treatment, to be executed simultaneously with the hyperthermia treatment. A hyperthermia treatment control module executes the hyperthermia therapy control data processed by the first process module; and a brachy therapy control module executes the brachy therapy control data for brachy therapy treatment simultaneous with the hyperthermia treatment control module.

While the device according to the invention may be manufactured by conventional methods, a practical manufacturing process may involve: providing a polymer tube; providing a mask material on the tube; defining an electrode structure in the mask material; providing an conductive material on the mask material so as to provide the conductive material on the tube in the electrode structure previously defined; and depositing a dielectric layer covering the conductive material, for example, after removing the mask material.

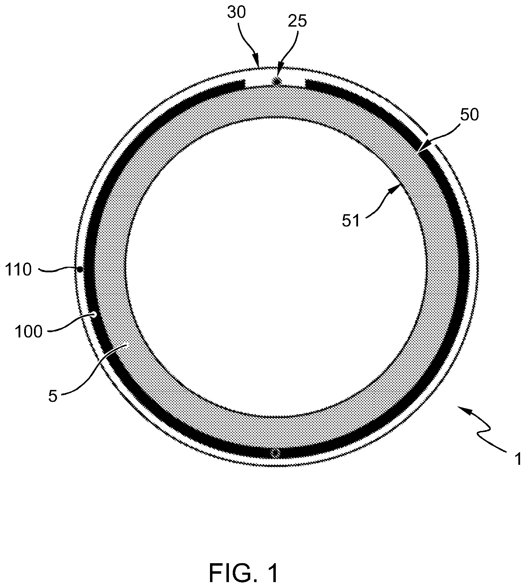

Now turning to Figure 1 there is shown a cross sectional view of an interstitial hyperthermia device 1, i.e. a direct capacitively coupled type interstitial hyperthermia-Brachytherapy applicator. Electrode structure 100 is deposited on a flexible prostate applicator device 1. The device 1 is provided with a hollow source guide 5 for conducting a radiation source capsule (not shown, conventionally known to the skilled person) to be moved by a guidewire. The hollow source guide 5 has an inner wall 51 for guiding the source capsule and an outer wall 50 to be contacted with the patient's tissue. The source guide 5 is typically of a polymer material, such as Polyoxymethylene (POM), or any suitable material to the purpose that is elegible for providing an electrode structure 100 arranged on a circumference of the outer wall 50 of the hollow source guide 5 and having a dielectric layer 110 shielding the electrode structure 100 from the patient's tissue. The dielectric layer 110 may be a Parylene layer or any suitable dielectric, e.g. a dielectric with dielectric constant ranging between 2 and 4, for example in range of 2.3 to 3.2 to cover the electrode structure 100. The dielectric material is chosen to have a suitable dielectric constant while having low a relatively low dissipation factor/loss tangent: i.e. less than 0.05 to prevent heating up of the catheter, which would result in heat up due to thermal conduction from the applicator's surface, instead of heat up due to power deposition by the alternating electric field in the patient's tissue. The dielectric material may cover the conductive structures and may extend to cover also the rest of the source guide catheter, while thin wires run through passage 30 to connect the electrodes to the current source. In the Figure there is shown a single electrode wire 25 axial passage 30, aligned with the tube 5, wherein an electrode wire 25 is provided running through said passage 30, that connects to another electrode of said plurality of electrodes - see subsequent figures. The thickness of the dielectric layer 110 is preferably less than 50 micrometer to ensure that the layer does not substantially heat up due to electric losses. In a preferred embodiment, the dielectric layer 110 is deposited by e.g. vapour deposition, to a thickness of 30 micrometer, which is a suitable thickness while ensuring no galvanic contact is possible with the patient's tissue. A minimum layer thickness could be about 10 micrometer.

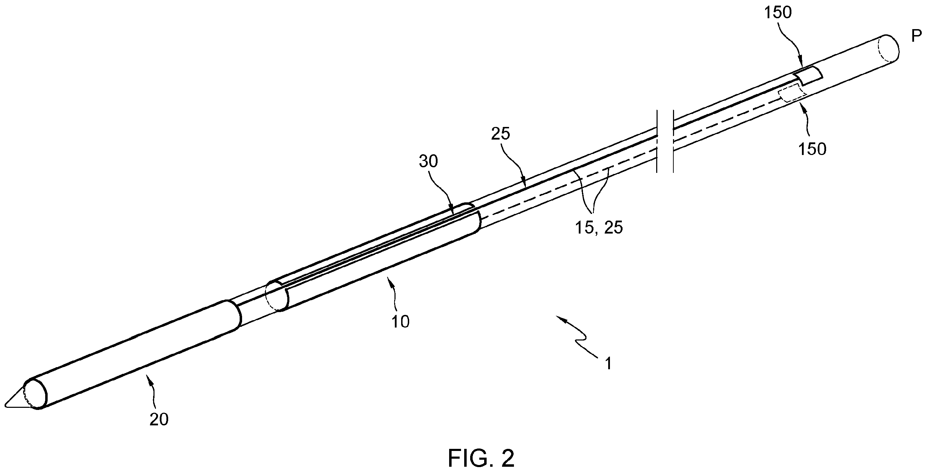

Figure 2 shows a perspective view of the hyperthermia device 1 of Figure 1, showing an electrode structure having two electrodes 10, 20, wherein the distal electrode 20 does not have a passage, and the proximal electrode 10 has a passage 30 through which electrode wire 25 connects electrode to a signal generator (not shown) on the proximal side P via connectors 150. In the shown example the applicator is a dual electrode (i.e. two electrodes 10, 20) applicator 1, that allows excellent longitudinal temperature control and temperature steering. The applicator 1 may be based on a (standard) 6F POM HDR-BT afterloading catheter having conductive electrodes of e.g 30 µm thickness and 20 mm (e.g.: 15-25 mm) length along the applicator 1. A minimal thickness of the electrode material would be about 15 micrometer, which is a thickness comparable to the skin-depth of copper at a driving frequency of 27 MHz, assuming a thickness accuracy of 50%, thereby providing a conductive layer thicker than the skin depth. In between the electrodes there can be provided a spacing of e.g. 5 mm. The two electrodes 10, 20 may be driven with a 120 µm wide line of equal thickness to the electrodes that ends up in the proximal part of the tube, where a connection patch 150 is positioned for connection to the RF power source. On top of the conductive electrode layer a dielectric layer of e.g. Parylene C or any other suitable biocompatible dielectric material is deposited to ensure direct capacitive coupling with the tissue, while maintaining biocompatibility.

While Figure 2 shows a pair of electrodes 10, 20 the number of electrodes may be any number ranging from 1 to as many are feasible for designing on a catheter tube. In a 2D geometry with passage lines, as will be illustrated below, the passage 30 may, with increasing number of electrode wires eventually break the circumferential homogeneity of the temperature, since the capacitive coupling effect will be reduced near passage 30.

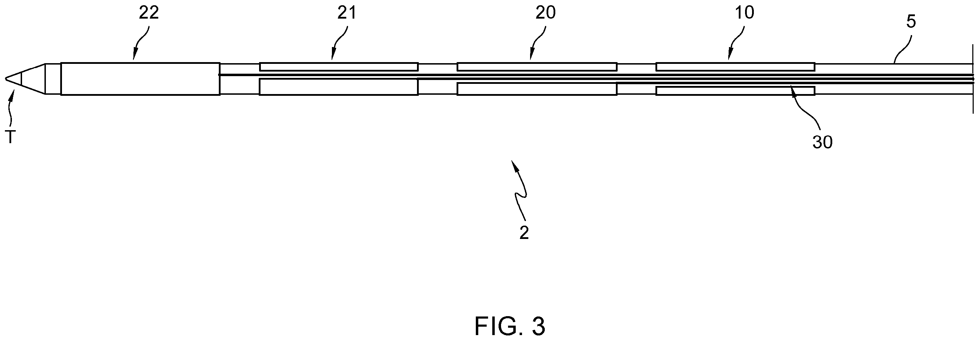

Figure 3 shows that at least four electrodes may be present on hyperthermia device 2, where a proximal electrode 10 allows passage to the three electrode wires of electrodes 20, 21 and distal electrode 22 provided near the (distal) tip T of the tube 5. In a further increasing number of electrodes, the electrodes may be connected by electrode wires that are provided in a 3D configuration, e.g. with a grid layer and a via structure, manufacturable with the same principles as the methods sketched below.

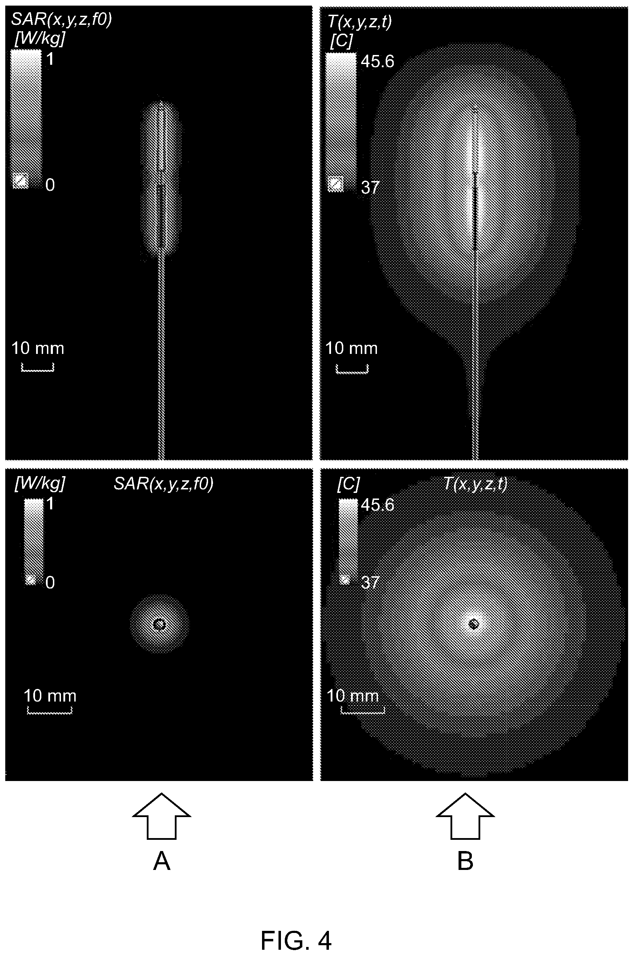

Figure 4 shows electromagnetic and heating simulation results of a single applicator: in Figure 4a the Specific Absorption Rate (SAR) distributions; in Figure 4b the temperature distributions after 20 minutes of heating with 1 W of irradiated power (right) in the horizontal plane passing through the center of the applicator axis (top) and in the plane perpendicular to the center of the bottom electrode (bottom).

Noticeable is that the device provides a deep SAR and temperature penetration; where the penetration depth is the radial depth where the SAR or Temperature is reduced to 1/e of the maximum SAR. The applicator can have a SAR penetration depth of about 2.2 mm which is superior to other techniques which usually show a 1/r² SAR falloff, which leads to a penetration depth of 1.7 mm. The penetration depth for temperature is 9.5 mm in a homogenous perfused muscle equivalent tissue.

The applicator also provides a very efficient power delivery to the tissue since, due to the thin dielectric layer between the electrodes and the tissue, less power is deposited inside the catheter wall. Self-heating of the applicator is therefore considerably reduced and thereby the absorption of the electromagnetic energy becomes the dominant factor governing the temperature pattern. This is in contrast to thermal conduction which is the predominant factor in conventional interstitial hyperthermia devices. The fraction of the total radial SAR inside the applicator/catheter 1 may be smaller than 2%, which is a considerable gain relative to conventional applicators that operate with electrodes inside a tube and may have dielectric losses as large as 33% or higher.

With electrodes on the outside of the IHT applicator, there is only low power deposition inside the catheter. The temperature increase in the tissue is caused by direct power deposition rather than thermal conduction from an overheated applicator/catheter when IHT heating source would be placed inside the catheter. Therefore, changing the power delivered to the electrode leads to a more rapid change in tissue temperature.

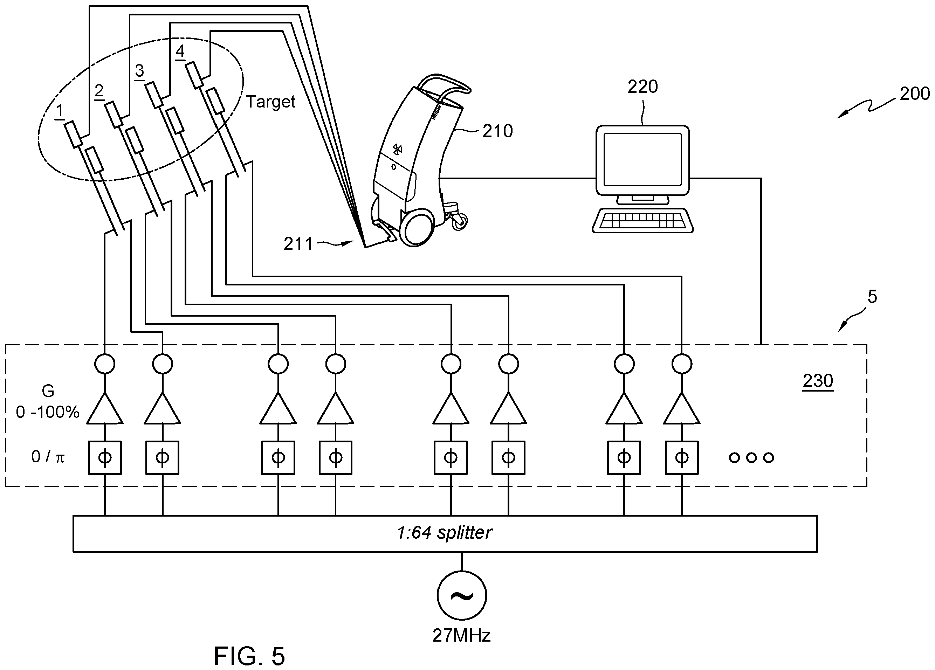

Figure 5 shows an interstitial hyperthermia control system for driving multiple interstitial devices simultaneously. While many electrode configurations and driving options are possible, advantageously, the control system is equipped to provide temperature steering by driving one or more electrodes in the electrode structure of one or more of the hyperthermia devices, to create a three dimensional heat profile in the patient's tissue.

In the figure, control system 200 is provided by a set of a conventional afterloading device known in the art as a brachytherapy afterloader 210 and an interstitial device control system 220, equipped to drive signal generator 230, based on an effective dose calculation, that is coupled to respective electrodes of hyperthermia applicator devices 1-4. The drive signal generator 230 is programmed to drive one or more electrodes 1-4 in the electrode structure of one or more of the hyperthermia devices, to create a three-dimensional heat profile in the patient's tissue. In the Figure, interstitial applicators 1 -4 are shown for guiding a radiation source in a human body part for brachytherapy applications. The interstitial devices 1-4 are to be connected to after loader 210, in which radiation sources can be stored, if not in use. In use, the radiation source is taken from the housing 210 and emits radiation in the patient's tissue, by guiding the source via a guidewire, which guides the source into and out of the housing 210 by means of a movement mechanism, via guide tubes 211. The guide tube 12 connects to an interstitial hyperthermia device 14 inserted into the body 4, to the positions to be irradiated in the body.

The system 200 defines a target area for the treatment of tissues in the human body. In this treatment area, predetermined positions, the so-called dwell positions, are taken by a radiation source traveling from the afterloader 210 into interstitial devices 1-4 to achieve an optimal irradiation result for the body according to a predetermined time schedule. To this end, the system 200, in particular afterloader 210 can keep a radiation source in a number of fixed, presettable e.g. programmable positions in the target area, which are, for instance, always spaced 1 mm apart, while a movement mechanism comprises, for instance, a stepping motor to guide a radiation source through the applicator in a stepwise manner by moving the source capsule through the interstitial hyperthermia devices 1-4.

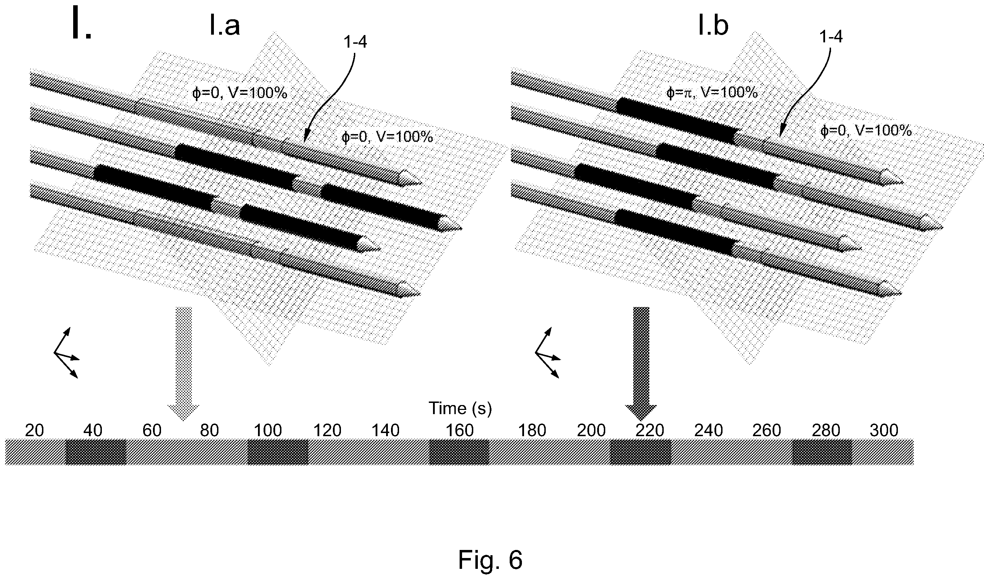

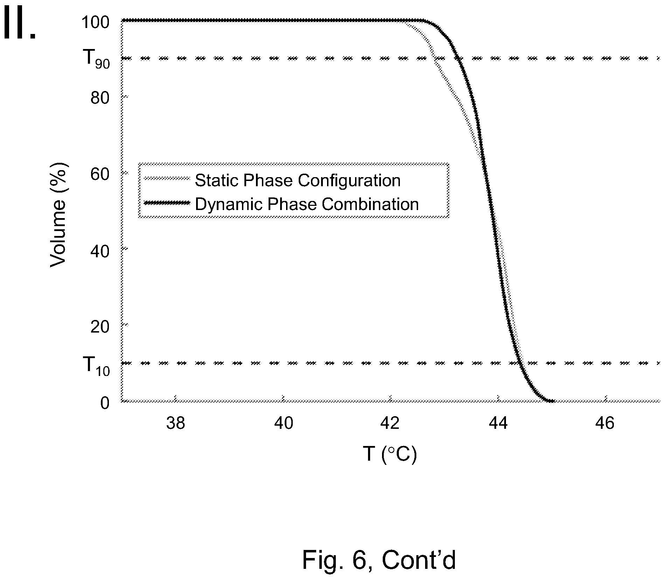

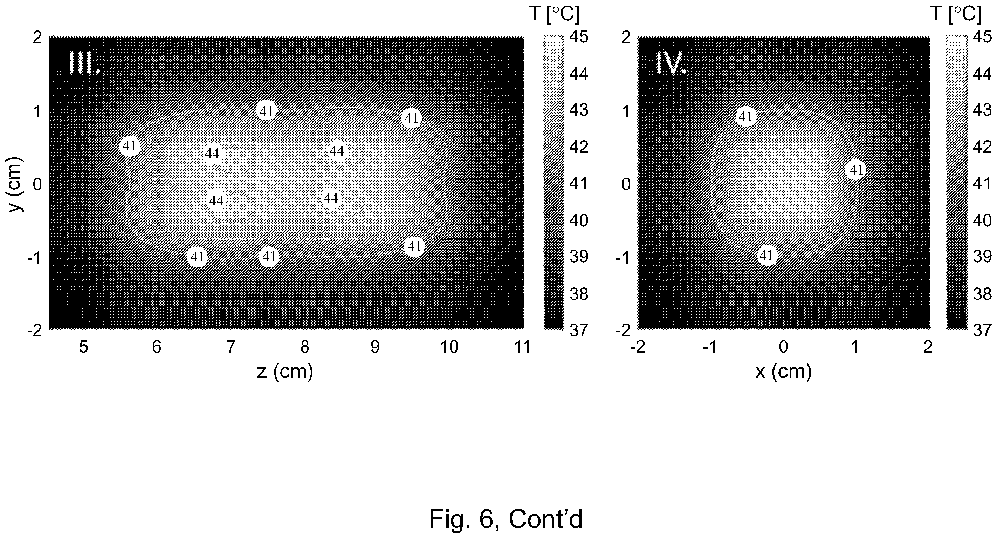

Drive control system 230, in addition to providing driving signals to the electrodes, also controls their respective phases as will be demonstrated in the example of Figure 6. The driving power control system can provides independent control of the power gain and phase of each electrode at a central driving frequency of for example 27 MHz, or any suitable driving frequency between 10 and 50 MHz that is capable of heating up the tissue. While capacitive coupling is preferably provided by electrodes in antiphase, any suitable phase difference may have a heating effect.

The drive control system 230 may thus function simultaneously to the HDR-Brachytherapy treatment by having the applicators 1-4 connected to afterloader 210. Compared to a standard 6F catheter the applicators 1-4 are almost equal in thickness and will not require modifications on the existing remote afterloader technology (e.g. 2.12 mm compared to 2.00 mm is a 6% increase in diameter). The metallic layers of the applicator are very thin and do not disturb the radiation emitted from the BT source inside the applicator (e.g. <0.05% beam intensity reduction).

Figure 6 shows a temperature profile of an electrode structure in a number of simultaneously driven phase control modes e.g. provided by drive control system 230. Through different phase control modes of the various applicators 1-4 that are used, more degrees of freedom can be provided in volumetric temperature steering, i.e. providing a certain desired temperature profile. In Figure 6 simulated data are shown of a temperature profile after 5 minutes of heating in an agar phantom with dynamic combination of two phase configurations illustrated in Fig 6 I.a and I.b. In Fig 6 II a cumulative temperature volume histogram comparison is shown between the static phase configuration and the dynamic phase configuration in a volume of interest presented in the temperature maps (Figs 6 III and 6 IV). Fig 6 III shows a central plane parallel to the applicators; Fig 6 IV shows a plane transverse to the applicators in the center of the active length of the applicators.

Figure 6 I shows an electrode structure having two electrodes arranged in axial direction along the source guide of interstitial devices 1-4. The power gain and polarity of each electrode can be adapted during the hyperthermia treatment, allowing dynamic adaptation during treatment. This configuration allows the power distribution to switch periodically between different configurations which, combined, can produce more temperature patterns.

For example, in Figure 6 Ia, each electrode of devices 1-4 is controlled with drive signals with no mutual phase difference; while the electrodes of hyperthermia devices 1-4 are driven in antiphase relative to each other, when positioned adjacent to each other and separated from each other, thus providing a capacitive coupling effect between adjacent electrodes. Alternatively in Figure 6 I.b the electrodes may be driven each with a mutual phase difference e.g. of 180 degrees establishing a capacitive coupling effect between the electrodes of a single device. The electrodes can thus be controlled to provide a length of a temperature profile along the applicator that enables a fully personalized design of treatment, by phase control.

Besides configuration where electrodes are driven in antiphase relative to each other, also the electrode length can be further adapted, leading to an even more personalized thermal treatment. The freedom of heating length adaptation is a unique feature. It comes in contrast to MW techniques where the shape of the temperature distribution is frequency dependent.

The described design provides improved temperature homogeneity in the target tissue of a patient, since the independently controlled electrodes per applicator may provide longitudinal temperature control and temperature homogeneity even in targets with an inhomogeneous blood perfusion. Furthermore, due to the dynamic adaptability of the phase and amplitude of each individual electrode, the electrodes can change their phase and amplitude periodically to achieve a more homogeneous temperature distribution. For example a heat profile can be shifted axially by careful timing of the electrodes, e.g. if a heat profile is desired proximal to the applicator, the distal electrodes may be silenced and vice versa, if a heat profile is desired more central, the electrodes of each applicator may be driven in antiphase. The applicators can thus be controlled to change the heating location to adapt to anatomic and physiologic changes in the heating region. This contrast with systems where only amplitude adaptation is possible, or with systems where multiple heating elements are controlled by the same channel.

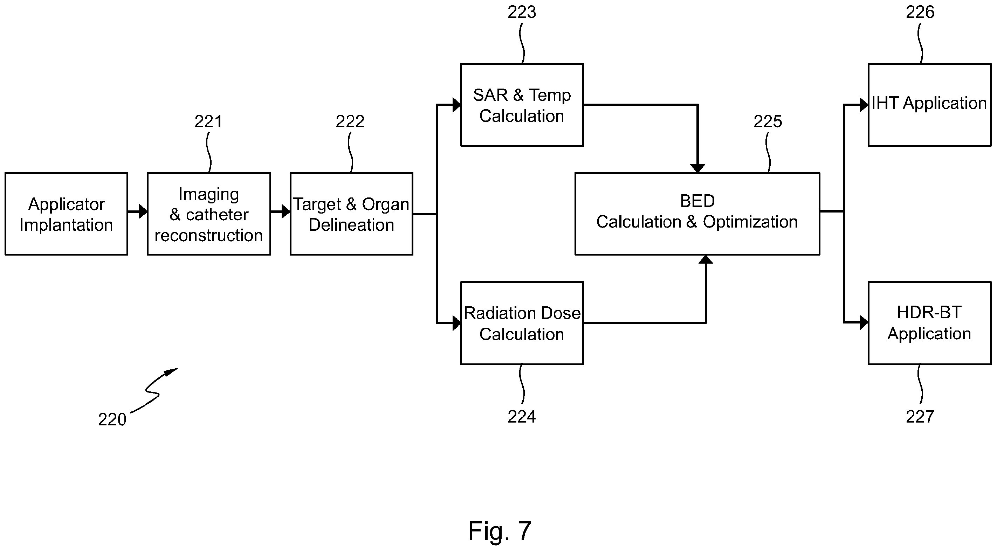

Figure 7 shows an interstitial hyperthermia control system 220 in more detail, e.g. comprising a treatment and a treatment planning module 225 for combined treatment of hyperthermia and brachytherapy. In the further treatment planning module 225 a Biological Effective Dose is calculated for combined treatment of hyperthermia and brachytherapy, rather than a standalone Interstitial Hyperthermia treatment planning or a Brachytherapy planning; since the biological effective dose is dependent on both radiation dosage and target tissue temperature. The novel ThermoBrachytherapy system can thereby offer a great clinical and economic advantage of seamless integration of the IHT radiosensitization method in an already established HDR-BT routine. Moreover, no extra implants are needed due to the dual functionality of the ThermoBrachytherapy applicators. The simultaneous application of the two modalities provides the shortest treatment time possible without any relevant additional discomfort for the patient.

Further exemplary elements of the control system 220 include an image module 221; arranged to receive an image of the implanted interstitial hyperthermia device in the patient's tissue to be treated. A target and organ delineation module 222 is arranged to identify a treatment target and organs at risk in the received image. A first process module 223 is programmed for processing hyperthermia therapy control data in accordance with a treatment plan, and said identified treatment targets and organs at risk identified from the organ delineation module 222. A second process module 224 is programmed for controlling brachy therapy control data for brachy therapy treatment. The BED calculation and optimization module 225 optimizes the control data of modules 223 and 224 for simultaneous execution. A hyperthermia treatment control module 226 is programmed for executing the hyperthermia therapy control data processed by the first process module 223 and the optimization module 225; and a brachy therapy control module 227 is programmed for executing the brachy therapy control data for brachy therapy treatment simultaneous with the hyperthermia treatment control module 226.

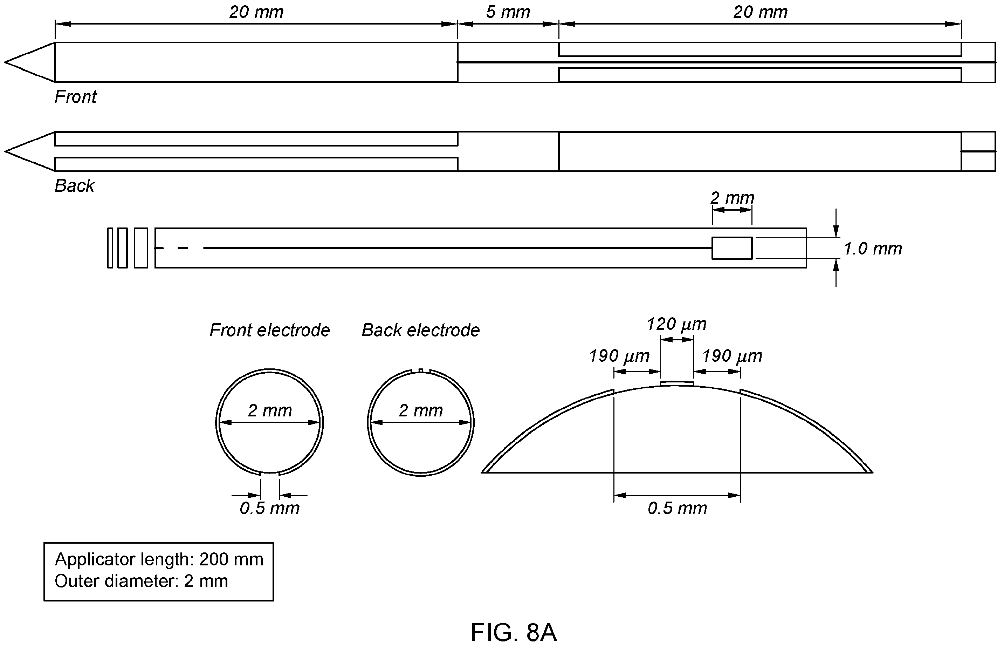

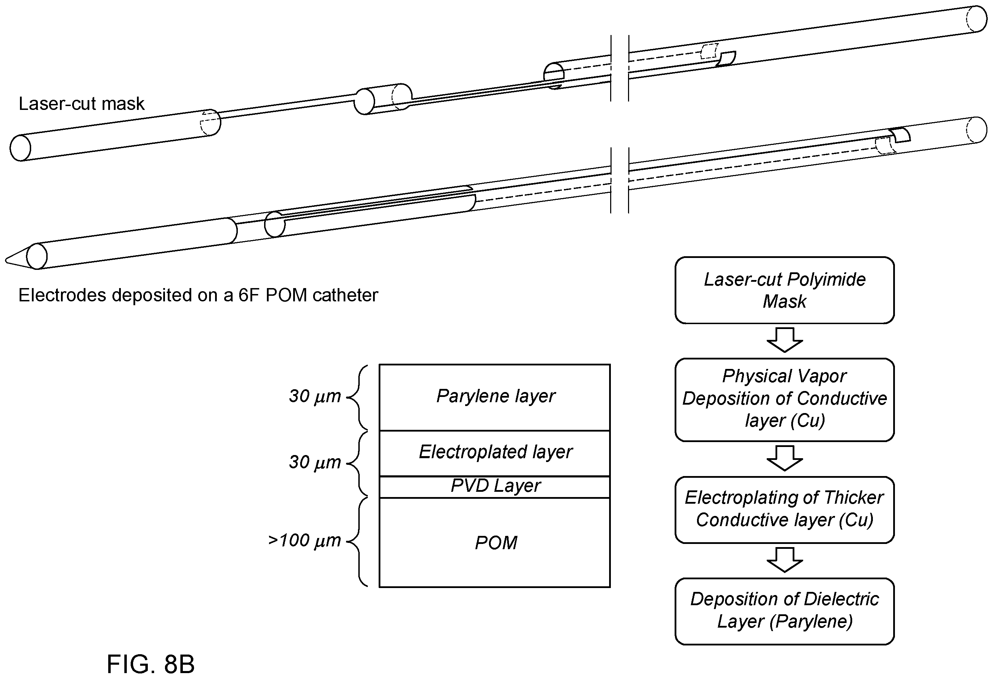

Figure 8 shows a further detail of an exemplary embodiment that shows, without being specifically limited to these dimensions, two 30 µm thick and 20 mm long copper layers that are deposited onto the outer layer of a 6 Fr HDR-BT flexible POM afterloading catheter, with a spacing of 5 mm between the electrodes. The copper layers leave a 0.5 mm azimuthal opening along the catheter axis, which serves as a wiring path. The copper electrodes are connected by a copper line (120 µm width) to the posterior side of the catheter, from where they can be connected to the source. A thin dielectric coating layer (30 µm Parylene C) covers the outer applicator to ensure capacitive coupling rather than galvanic contact with the tissue and to ensure biocompatibility. The applicator can be fabricated using a lithography based deposition method: First a polyimide tube with an inner diameter of 2 mm is laser-cut in the negative shape of the desired conductive layer shape. The polyimide tube functions as a mask for the deposition of the conductive layer on a POM catheter, used for Brachytherapy. Then, a thin conductive layer is deposited on the masked POM catheter using Physical Vapor Deposition. After that, the conductive layer is brought to a desired thickness of e.g. 30 mircrometer using electroplating. Finally, the catheter is covered with a Parylene layer of a similar dimension of e.g. 10-50 µm, e.g. also by vapour deposition.

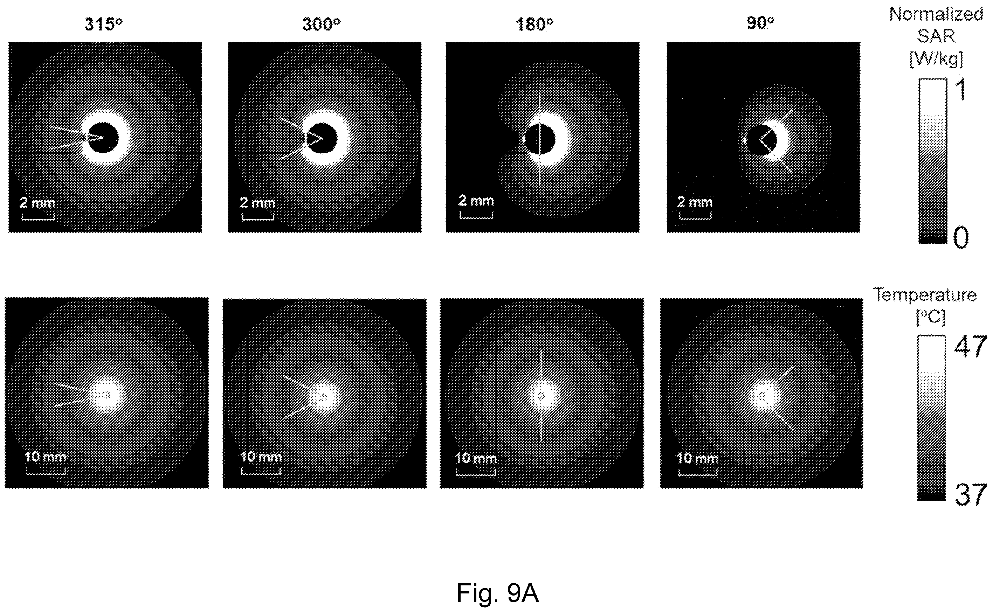

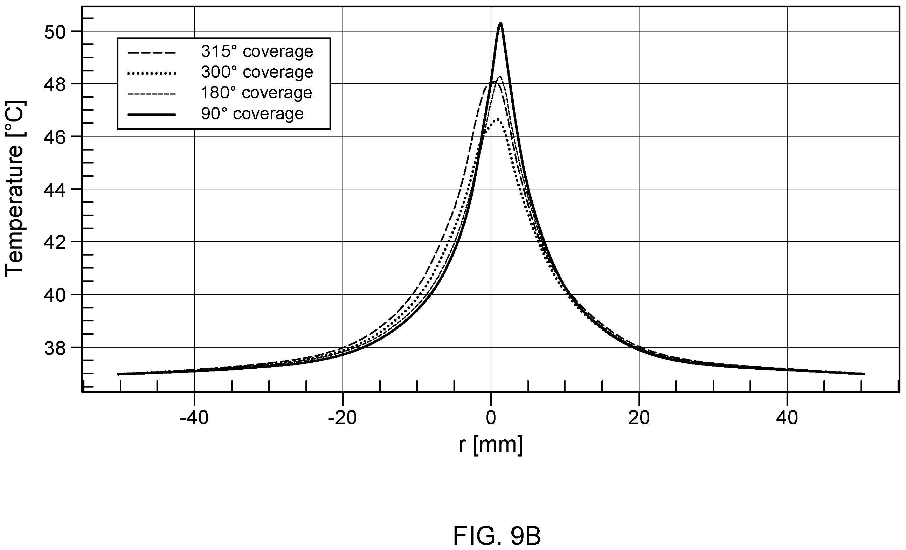

Figure 9A and B show examples of an alternative embodiment, having an asymmetric temperature profile. More specifically Figure 9A shows simulated normalized Specific Absorption Rate (SAR) and Temperature distribution plots after 20min of heating with 1 W of input power in an applicator inserted in a perfused muscle equivalent tissue. The 2D distributions are on the central axis of a 10 mm long electrode segment covering (from left to right) 315, 300, 180 or 90 degrees of the cylindrical surface of the applicator. An electrode wire line transfers power to a second electrode passes through the center of the uncovered surface, indicated as 30 in Figure 1. Lines A represent the radial limits of the electrodes. Depending on the axial passage width, for transferring the electrode wire structure past the electrode, the circumferential covering of an electrode may vary, e.g. from about 315 degrees (single wire) to even 90 degrees, allowing passage for more than two separate electrodes. As is seen in Figure 9A the difference between an electrode covering 315 degrees (needed for a dual electrode applicator) and 300 degrees (needed for a triple electrode applicator) of the cylindrical radius does not result in significant changes in the radial temperature distribution. Figure 9B specifically shows a simulated temperature distribution after 20 min of heating with 1W of input power in an applicator inserted in a perfused muscle equivalent tissue in a different manner. The r axis of Figure 9B passes through the center of the applicator and through the radial center of the electrode segment. The different lines refer to different radial coverages of the electrode. In general, a smaller radial coverage of the electrode may lead to a significant shift of the heat distribution peak away from the center of the applicator e.g, a distance where the temperature crosses the 44°C is on the non-electrode side about 2.5 mm whereas is at the electrode covered side about 5.0 mm. Hence, a doubling of the distance for the high temperature. This can be a critical contribution for the exploitation of the thermal enhancement effect in the tumor while at the same time protecting an organ at risk, i.e. lower temperature. As can be seen in Figure 9b that makes the temperature distribution more heterogeneous.

It is thus believed that the operation and construction of the present invention will be apparent from the foregoing description and drawings appended thereto. For the purpose of clarity and a concise description, features are described herein as part of the same or separate embodiments, however, it will be appreciated that the scope of the invention may include embodiments having combinations of all or some of the features described.

The system could be applied wherever interstitial HDR-BT is considered a beneficial procedure to apply ionising radiation in the process of cancer treatment. Among others: Prostate Cancer, Glioblastoma Multiforme cancer treatment, Gynaecological cancer treatment, Rectal cancer treatment, Breast cancer treatment, Head & Neck cancer treatment, etc.

With the electrode structure arranged on an outer wall of the hollow source guide there may be small extra layers but in essence, the only outer layer will be the thin dielectric layer. The hollow source guide will preferably be tubular, but this does not exclude other forms. Also it's outer circumference will be preferably tubular, but also other cross sections, i.e. square or triangular cross sections may be used. It will be clear to the skilled person that the invention is not limited to any embodiment herein described and that modifications are possible which may be considered within the scope of the appended claims. Also kinematic inversions are considered inherently disclosed and can be within the scope of the invention. In the claims, any reference signs shall not be construed as limiting the claim. The terms 'comprising' and 'including' when used in this description or the appended claims should not be construed in an exclusive or exhaustive sense but rather in an inclusive sense. Thus expression as 'including' or 'comprising' as used herein does not exclude the presence of other elements, additional structure or additional acts or steps in addition to those listed. Furthermore, the words 'a' and 'an' shall not be construed as limited to 'only one', but instead are used to mean 'at least one', and do not exclude a plurality. Features that are not specifically or explicitly described or claimed may additionally be included in the structure of the invention without departing from its scope. Expressions such as: "means for ..." should be read as: "component configured for ..." or "member constructed to ..." and should be construed to include equivalents for the structures disclosed. The use of expressions like: "critical", "preferred", "especially preferred" etc. is not intended to limit the invention. To the extent that structure, material, or acts are considered to be essential they are inexpressively indicated as such. Additions, deletions, and modifications within the purview of the skilled person may generally be made without departing from the scope of the invention, as determined by the claims.

## Claims

1. An interstitial hyperthermia device (1) having an electrode structure (100) to be coupled to an electric power source for providing an alternating electric field for heating up a patient's tissue; said device provided with a hollow source guide (5) for conducting a radiation source capsule to be moved by a guidewire; said hollow source guide having an inner wall (51) for guiding the source capsule and an outer wall (50) to be contacted with the patient's tissue; **characterised in that** the outer wall being provided with the electrode structure arranged on the outer wall of the hollow source guide and having a dielectric coating layer (110) shielding the electrode structure from the patient's tissue.

2. The interstitial hyperthermia device according to claim 1, wherein the electrode structure is arranged on a polymer tube.

3. The interstitial hyperthermia device according to any preceding claim, wherein the dielectric layer has a thickness ranging between 20 and 40 micrometers.

4. The interstitial hyperthermia device according to claim 1 wherein said electrode structure comprises a plurality of electrodes arranged in axial direction along the source guide.

5. The interstitial hyperthermia device according to claim 4, wherein at least one electrode of said plurality of electrodes is provided with an axial passage, aligned with the tube.

6. The interstitial hyperthermia device according to claim 5, wherein said passage allows one or more wires to connect to more than one electrode of said plurality of electrodes.

7. The interstitial hyperthermia device according any preceding claim, wherein the dielectric coating layer is a Parylene layer.

8. An interstitial hyperthermia control system (200), comprising one or more of the hyperthermia devices (1) according to any previous claims, said control system programmed to drive one or more electrodes in the electrode structure of one or more of the hyperthermia devices, to create a three-dimensional heat profile in the patient's tissue.

9. The interstitial hyperthermia control system according to claim 8 programmed to drive said one or more electrodes in an electrode structure of one hyperthermia device in antiphase relative to one or more electrodes in an electrode structure of another hyperthermia device, when positioned adjacent each other and separated from each other.

10. The interstitial hyperthermia control system according to claim 8 when combined with claim 4, wherein the plurality of electrodes arranged in axial direction other along the source guide are driven in antiphase.

11. The interstitial hyperthermia control system according to any preceding claim 8-10, further comprising a treatment and a treatment planning module (225) calculating a Biological Effective Dose for combined treatment of hyperthermia and brachytherapy.

12. The interstitial hyperthermia control system according to claim 11, wherein the treatment planning module is provided with:
- an image module (221), arranged to receive an image of the implanted interstitial hyperthermia device in the patient's tissue to be treated;
- a target and organ delineation module (222), arranged to identify a treatment target and organs at risk in the received image;
- a first process module (223) for processing hyperthermia therapy control data in accordance with a treatment plan, and said identified treatment targets and organs at risk;
- a second process module (224) for controlling brachy therapy control data for brachy therapy treatment, to be executed simultaneously with the hyperthermia treatment;
- a hyperthermia treatment control module (226) for executing the hyperthermia therapy control data processed by the first process module; and
- a brachy therapy control module (227) for executing the brachy therapy control data for brachy therapy treatment simultaneous with the hyperthermia treatment control module.

13. A method of creating the interstitial hyperthermia device (1) of any preceding claim, comprising:
- providing a polymer tube;
- providing a mask material on the tube;
- defining an electrode structure (100) in the mask material;
- providing an conductive material on the mask material so as to provide the conductive material on the tube in the electrode structure previously defined;
- depositing a dielectric layer (110) covering the conductive material.

## Patentansprüche

1. Vorrichtung (1) für interstitielle Hyperthermie, aufweisend eine Elektrodenstruktur (100), die dazu vorgesehen ist, an eine elektrische Stromquelle gekoppelt zu werden, um ein elektrisches Wechselfeld zum Erwärmen von Gewebe eines Patienten bereitzustellen;
wobei die Vorrichtung mit einer hohlen Quellenführung (5) zum Führen einer von einem Führungsdraht zu führenden Strahlungsquellenkapsel bereitgestellt ist;
wobei die hohle Quellenführung eine Innenwand (51) zum Führen der Quellenkapsel und eine Außenwand (50) aufweist, die dazu vorgesehen ist, mit dem Gewebe des Patienten in Kontakt gebracht zu werden;
**dadurch gekennzeichnet, dass** die Außenwand derart bereitgestellt ist, dass die Elektrodenstruktur auf der Außenwand der hohlen Quellenführung angeordnet ist und eine dielektrische Beschichtung (110) aufweist, welche die Elektrodenstruktur gegen das Gewebe des Patienten abschirmt.

2. Vorrichtung für interstitielle Hyperthermie nach Anspruch 1, wobei die Elektrodenstruktur auf einer Polymerröhre angeordnet ist.

3. Vorrichtung für interstitielle Hyperthermie nach einem der vorhergehenden Ansprüche, wobei die dielektrische Beschichtung eine Dicke im Bereich zwischen 20 und 40 Mikrometern aufweist.

4. Vorrichtung für interstitielle Hyperthermie nach Anspruch 1, wobei die Elektrodenstruktur eine Vielzahl von Elektroden umfasst, die in axialer Richtung entlang der Quellenführung angeordnet sind.

5. Vorrichtung für interstitielle Hyperthermie nach Anspruch 4, wobei mindestens eine Elektrode der Vielzahl von Elektroden mit einer axialen Durchführung bereitgestellt ist, die mit der Röhre ausgerichtet ist.

6. Vorrichtung für interstitielle Hyperthermie nach Anspruch 5, wobei die Durchführung es ermöglicht, ein oder mehrere Drähte mit mehr als einer Elektrode der Vielzahl von Elektroden zu verbinden.

7. Vorrichtung für interstitielle Hyperthermie nach einem der vorhergehenden Ansprüche, wobei die dielektrische Beschichtung eine Parylenschicht ist.

8. Steuersystem (200) für interstitielle Hyperthermie, aufweisend eine oder mehrere der Hyperthermievorrichtungen (1) nach einem der vorherigen Ansprüche, wobei das Steuersystem dazu programmiert ist, ein oder mehrere Elektroden in der Elektrodenstruktur einer oder mehrerer der Hyperthermievorrichtungen anzusteuern, um ein dreidimensionales Wärmeprofil in dem Gewebe des Patienten zu erzeugen.

9. Steuersystem für interstitielle Hyperthermie nach Anspruch 8, das dazu programmiert ist, die eine oder die mehreren Elektroden in einer Elektrodenstruktur einer Hyperthermievorrichtung in Gegenphase relativ zu einer oder mehreren Elektroden in einer Elektrodenstruktur einer anderen Hyperthermievorrichtung anzusteuern, wenn sie nebeneinander liegen und voneinander getrennt sind.

10. Steuersystem für interstitielle Hyperthermie nach Anspruch 8 kombiniert mit Anspruch 4, wobei die Vielzahl von Elektroden, die in Axialrichtung entlang der Quellenführung angeordnet sind, gegenphasig angesteuert werden.

11. Steuersystem für interstitielle Hyperthermie nach einem der vorherigen Ansprüche 8 bis 10, ferner umfassend eine Behandlung und ein Behandlungsplanungsmodul (225), welches eine biologisch wirksame Dosis für eine kombinierte Behandlung mit Hyperthermie und Brachytherapie berechnet.

12. Steuersystem für interstitielle Hyperthermie nach Anspruch 11, wobei das Behandlungsplanungsmodul bereitgestellt ist mit:
- einem Bildmodul (221), das dazu angeordnet ist, ein Bild der in das zu behandelnde Gewebe des Patienten implantierten Vorrichtung für interstitielle Hyperthermie zu empfangen;
- einem Ziel- und Organkonturierungsmodul (222), das dazu angeordnet ist, ein Behandlungsziel und gefährdete Organe in dem empfangenen Bild zu identifizieren;
- einem ersten Prozessmodul (223) zum Verarbeiten von Hyperthermietherapie-Steuerdaten gemäß einem Behandlungsplan und den identifizierten Behandlungszielen und gefährdeten Organen;
- einem zweiten Prozessmodul (224) zum Steuern von Brachytherapie-Steuerdaten für eine gleichzeitig mit der Hyperthermiebehandlung auszuführende Brachytherapiebehandlung;
- einem Hyperthermiebehandlung-Steuermodul (226) zum Ausführen der von dem ersten Prozessmodul verarbeiteten Hyperthermietherapie-Steuerdaten; und
- einem Brachytherapie-Steuermodul (227) zum Ausführen der Brachytherapie-Steuerdaten zur Brachytherapiebehandlung gleichzeitig mit dem Hyperthermiebehandlung-Steuermodul.

13. Verfahren zum Herstellen der Vorrichtung (1) für interstitielle Hyperthermie nach einem der vorherigen Ansprüche, umfassend:
- Bereitstellen einer Polymerröhre;
- Bereitstellen eines Maskenmaterials auf der Röhre;
- Definieren einer Elektrodenstruktur (100) in dem Maskenmaterial;
- Bereitstellen eines leitfähigen Materials auf dem Maskenmaterial dergestalt, dass das leitfähige Material auf der Röhre in der zuvor definierten Elektrodenstruktur bereitgestellt wird;
- Abscheiden einer dielektrischen Schicht (110), die das leitfähige Material bedeckt.

## Revendications

1. Dispositif d'hyperthermie interstitielle (1) comportant une structure d'électrode (100) destinée à être couplée à une source d'énergie électrique pour produire un champ électrique alternatif destiné à chauffer les tissus d'un patient ; ledit dispositif étant équipé d'un guide de source creux (5) pour guider une capsule de source de rayonnement déplacée par un fil-guide ; ledit guide de source creux comportant une paroi interne (51) pour guider la capsule de source et une paroi externe (50) destinée à être en contact avec les tissus du patient ;
**caractérisé en ce que** la paroi externe est pourvue de la structure d'électrode disposée sur la paroi externe du guide de source creux et ayant une couche de revêtement diélectrique (110) protégeant la structure d'électrode des tissus du patient.

2. Dispositif d'hyperthermie interstitielle selon la revendication 1, dans lequel la structure d'électrode est disposée sur un tube polymère.

3. Dispositif d'hyperthermie interstitielle selon l'une quelconque des revendications précédentes, dans lequel la couche diélectrique a une épaisseur comprise entre 20 et 40 micromètres.

4. Dispositif d'hyperthermie interstitielle selon la revendication 1, dans lequel ladite structure d'électrodes comprend une pluralité d'électrodes disposées axialement le long du guide de source.

5. Dispositif d'hyperthermie interstitielle selon la revendication 4, dans lequel au moins une électrode de ladite pluralité d'électrodes est dotée d'un passage axial, aligné avec le tube.

6. Dispositif d'hyperthermie interstitielle selon la revendication 5, dans lequel ledit passage permet à un ou plusieurs fils de se connecter à plusieurs électrodes de ladite pluralité d'électrodes.

7. Dispositif d'hyperthermie interstitielle selon l'une quelconque des revendications précédentes, dans lequel la couche de revêtement diélectrique est une couche de Parylène.

8. Système de commande d'hyperthermie interstitielle (200), comprenant un ou plusieurs dispositifs d'hyperthermie selon l'une quelconque des revendications précédentes, ledit système de commande étant programmé pour piloter une ou plusieurs électrodes dans la structure d'électrodes d'un ou plusieurs dispositifs d'hyperthermie, afin de créer un profil thermique tridimensionnel dans les tissus du patient.

9. Système de commande d'hyperthermie interstitielle selon la revendication 8, programmé pour entraîner lesdites une ou plusieurs électrodes dans une structure d'électrode d'un dispositif d'hyperthermie en antiphase par rapport à une ou plusieurs électrodes dans une structure d'électrode d'un autre dispositif d'hyperthermie, lorsqu'elles sont positionnées adjacentes les unes aux autres et séparées les unes des autres.

10. Système de commande de l'hyperthermie interstitielle selon la revendication 8, lorsqu'elle est combinée à la revendication 4, dans lequel les électrodes disposées axialement le long du guide source sont commandées en opposition de phase.

11. Système de commande de l'hyperthermie interstitielle selon l'une quelconque des revendications 8 à 10, comprenant en outre un module de traitement et un module de planification de traitement (225) calculant une dose biologique efficace pour un traitement combiné par hyperthermie et curiethérapie.

12. Système de commande de l'hyperthermie interstitielle selon la revendication 11, dans lequel le module de planification de traitement comprend :
- un module d'imagerie (221), conçu pour recevoir une image du dispositif d'hyperthermie interstitielle implanté dans les tissus du patient à traiter ;
- un module de délimitation de cible et d'organe (222), conçu pour identifier une cible de traitement et les organes à risque sur l'image reçue ;
- un premier module de traitement (223) pour traiter les données de commande de la curiethérapie conformément au plan de traitement, aux cibles de traitement identifiées et aux organes à risque ;
- un deuxième module de traitement (224) pour commander les données de commande de la curiethérapie, à exécuter simultanément avec le traitement par hyperthermie ;
- un module de commande du traitement par hyperthermie (226) pour exécuter les données de commande de la curiethérapie traitées par le premier module de traitement ; et
- un module de commande de la curiethérapie (227) pour exécuter les données de commande de la curiethérapie simultanément avec le module de commande du traitement par hyperthermie.

13. Procédé de création du dispositif d'hyperthermie interstitielle (1) selon l'une quelconque des revendications précédentes, comprenant :
- la fourniture d'un tube polymère ;
- la fourniture d'un matériau de masque sur le tube ;
- la définition d'une structure d'électrode (100) dans le matériau de masque ;
- la fourniture d'un matériau conducteur sur le matériau de masque afin de fournir le matériau conducteur sur le tube dans la structure d'électrode précédemment définie ;
- la déposition d'une couche diélectrique (110) recouvrant le matériau conducteur.
